# EUROPEAN PATENT APPLICATION

(11) **EP 3 690 027 A1**
(43) Date of publication of application: **05.08.2020**
(21) Application number: 18874429.6
(22) Date of filing: 22.05.2018
(51) Int. Cl.: C12N 1/21, C12P 13/08, C12N 15/64, C12N 15/69, C12R 1/01, C12R 1/645

(54) **RECOMBINANT BACTERIUM FOR PRODUCING L-LYSINE, CONSTRUCTION METHOD THEREOF, AND METHOD FOR PRODUCING L-LYSINE**

(30) Priority: 01.11.2017 CN 201711058221
(71) Applicant: Ningxia Eppen Biotech Co. Ltd, Ningxia 750100 (CN); Institute Of Microbiology, Chinese Academy Of Sciences, Beijing 100101 (CN)
(72) Inventor: WEN, Tingyi, Beijing 100085 (CN); ZHANG, Chen, Beijing 100085 (CN); SHANG, Xiuling, Beijing 100085 (CN); CHAI, Xin, Beijing 100085 (CN); ZHANG, Yun, Beijing 100085 (CN); LIU, Shuwen, Beijing 100085 (CN); WANG, Guoqiang, Beijing 100085 (CN); LI, Zhongcai, Beijing 100085 (CN)
(74) Representative: Kurig, Thomas
(86) International application number: PCT/CN2018/087894
(87) International publication number: WO 2019/085445

(57) **Abstract**

A recombinant bacterium for producing L-lysine, a construction method thereof, and a method for producing L-lysine by using the recombinant bacterium. The recombinant bacterium has increased expression and/or activity of asparaginase compared to a starting bacterium.

Sequence Listing

<110>  INSTITUTE OF MICROBIOLOGY, CHINESE ACADEMY OF SCIENCE
<120>  RECOMBINANT BACTERIUM CAPABLE OF PRODUCING L-LYSINE, CONSTRUCTION
METHOD THEREOF AND PRODUCTION METHOD OF L-LYSINE

<130>  170710CI

<141>  2017-11-01

<160>  57

<170>  SIPOSequenceListing 1.0

<210>  1
<211>  1638
<212>  DNA
<213>  Corynebacterium glutamicum

<400>  1
   ctgcgacagc atggaactca gtgcaatggc tgtaaggcct gcaccaacaa tgattgagcg      60
   aagctccaaa atgtcctccc cgggttgata ttagatttca taaatatact aaaaatcttg     120
   agagtttttc cgttgaaaac taaaaagctg ggaaggtgaa tcgaatttcg gggctttaaa     180
   gcaaaaatga acagcttggt ctatagtggc taggtaccct ttttgttttg gacacatgta     240
   gggtggccga aacaaagtaa taggacaaca acgctcgacc gcgattattt ttggagaatc     300
   atgacctcag catctgcccc aagctttaac cccggcaagg gtcccggctc agcagtcgga     360
   attgcccttt taggattcgg aacagtcggc actgaggtga tgcgtctgat gaccgagtac     420
   ggtgatgaac ttgcgcaccg cattggtggc ccactggagg ttcgtggcat tgctgcttct     480
   gatatctcaa agccacgtga aggcgttgca cctgagctgc tcactgagga cgcttttgca     540
   ctcatcgagc gcgaggatgt tgacatcgtc gttgaggtta tcggcggcat tgagtaccca     600
   cgtgaggtag ttctcgcagc tctgaaggcc ggcaagtctg ttgttaccgc caataaggct     660
   cttgttgcag ctcactctgc tgagcttgct gatgcagcgg aagccgcaaa cgttgacctg     720
   tacttcgagg ctgctgttgc aggcgcaatt ccagtggttg gcccactgcg tcgctccctg     780
   gctggcgatc agatccagtc tgtgatgggc atcgttaacg gcaccaccaa cttcatcttg     840
   gacgccatgg attccaccgg cgctgactat gcagattctt tggctgaggc aactcgtttg     900
   ggttacgccg aagctgatcc aactgcagac gtcgaaggcc atgacgccgc atccaaggct     960
   gcaattttgg catccatcgc tttccacacc cgtgttaccg cggatgatgt gtactgcgaa    1020
   ggtatcagca acatcagcgc tgccgacatt gaggcagcac agcaggcagg ccacaccatc    1080
   aagttgttgg ccatctgtga gaagttcacc aacaaggaag gaaagtcggc tatttctgct    1140
   cgcgtgcacc cgactctatt acctgtgtcc cacccactgg cgtcggtaaa caagtccttt    1200
   aatgcaatct ttgttgaagc agaagcagct ggtcgcctga tgttctacgg aaacggtgca    1260
   ggtggcgcgc caaccgcgtc tgctgtgctt ggcgacgtcg ttggtgccgc acgaaacaag    1320
   gtgcacggtg gccgtgctcc aggtgagtcc acctacgcta acctgccgat cgctgatttc    1380
   ggtgagacca ccactcgtta ccacctcgac atggatgtgg aagatcgcgt ggggggtttg    1440
   gctgaattgg ctagcctgtt ctctgagcaa ggaatctccc tgcgtacaat ccgacaggaa    1500
   gagcgcgatg atgatgcacg tctgatcgtg gtcacccact ctgcgctgga atctgatctt    1560
   tcccgcaccg ttgaactgct gaaggctaag cctgttgtta aggcaatcaa cagtgtgatc    1620
   cgcctcgaaa gggactaa                                                  1638

<210>  2
<211>  26
<212>  DNA
<213>  Artificial Sequence

<220>
<221>  prim_bind
<222>  (1)..(26)
<223>  hom-V59A upstream forward primer


<400>  2
gctctagaag ctgtttcaca atttct                                           26

<210>  3
<211>  19
<212>  DNA
<213>  Artificial Sequence

<220>
<221>  prim_bind
<222>  (1)..(19)
<223>  hom-V59A mutation site reverse primer


<400>  3
atatcagaag cagcaatgc                                                    19

<210>  4
<211>  19
<212>  DNA
<213>  Artificial Sequence

<220>
<221>  prim_bind
<222>  (1)..(19)
<223>  hom-V59A mutation site forward primer


<400>  4
gcattgctgc ttctgatat                                                    19

<210>  5
<211>  27
<212>  DNA
<213>  Artificial Sequence

<220>
<221>  prim_bind
<222>  (1)..(27)
<223>  hom-V59A downstream reverse primer


<400>  5
ccggaattcc caacaacttg atggtgt                                           27

<210>  6
<211>  19
<212>  DNA
<213>  Artificial Sequence

<220>
<221>  prim_bind
<222>  (1)..(19)
<223>  hom-V59A forward identified primer


<400>  6
tctacgttgt atctcgcac                                                    19

<210>  7
<211>  18
<212>  DNA
<213>  Artificial Sequence

<220>
<221>  prim_bind

<222>  (1)..(18)
<223>  hom-V59A reverse identifiedprimer

<400>  7
caggcgacca gctgcttc                                                    18

<210>  8
<211>  2280
<212>  DNA
<213>  Corynebacterium glutamicum

<400>  8
aaaaccgatg tttgattggg ggaatcgggg gttacgatac taggacgcag tgactgctat      60
cacccttggc ggtctcttgt tgaaaggaat aattactcta gtgtcgactc acacatcttc     120
aacgcttcca gcattcaaaa agatcttggt agcaaaccgc ggcgaaatcg cggtccgtgc     180
tttccgtgca gcactcgaaa ccggtgcagc cacggtagct atttaccccc gtgaagatcg     240
gggatcattc caccgctctt ttgcttctga agctgtccgc attggtaccg aaggctcacc     300
agtcaaggcg tacctggaca tcgatgaaat tatcggtgca gctaaaaaag ttaaagcaga     360
tgccatttac ccgggatacg gcttcctgtc tgaaaatgcc cagcttgccc gcgagtgtgc     420
ggaaaacggc attactttta ttggcccaac cccagaggtt cttgatctca ccggtgataa     480
gtctcgcgcg gtaaccgccg cgaagaaggc tggtctgcca gttttggcgg aatccacccc     540
gagcaaaaac atcgatgaga tcgttaaaag cgctgaaggc cagacttacc ccatctttgt     600
gaaggcagtt gccggtggtg gcggacgcgg tatgcgtttt gttgcttcac ctgatgagct     660
tcgcaaatta gcaacagaag catctcgtga agctgaagcg gctttcggcg atggcgcggt     720
atatgtcgaa cgtgctgtga ttaaccctca gcatattgaa gtgcagatcc ttggcgatca     780
cactggagaa gttgtacacc tttatgaacg tgactgctca ctgcagcgtc gtcaccaaaa     840
agttgtcgaa attgcgccag cacagcattt ggatccagaa ctgcgtgatc gcatttgtgc     900
ggatgcagta aagttctgcc gctccattgg ttaccagggc gcgggaaccg tggaattctt     960
ggtcgatgaa aagggcaacc acgtcttcat cgaaatgaac ccacgtatcc aggttgagca    1020
caccgtgact gaagaagtca ccgaggtgga cctggtgaag gcgcagatgc gcttggctgc    1080
tggtgcaacc ttgaaggaat tgggtctgac ccaagataag atcaagaccc acggtgcagc    1140
actgcagtgc cgcatcacca cggaagatcc aaacaacggc ttccgcccag ataccggaac    1200
tatcaccgcg taccgctcac caggcggagc tggcgttcgt cttgacggtg cagctcagct    1260
cggtggcgaa atcaccgcac actttgactc catgctggtg aaaatgacct gccgtggttc    1320
cgactttgaa actgctgttg ctcgtgcaca gcgcgcgttg gctgagttca ccgtgtctgg    1380
tgttgcaacc aacattggtt tcttgcgtgc gttgctgcgg gaagaggact tcacttccaa    1440
gcgcatcgcc accggattca ttgccgatca ctcgcacctc cttcaggctc cacctgctga    1500
tgatgagcag ggacgcatcc tggattactt ggcagatgtc accgtgaaca agcctcatgg    1560
tgtgcgtcca aaggatgttg cagctcctat cgataagctg cctaacatca aggatctgcc    1620
actgccacgc ggttcccgtg accgcctgaa gcagcttggc ccagccgcgt ttgctcgtga    1680
tctccgtgag caggacgcac tggcagttac tgataccacc ttccgcgatg cacaccagtc    1740
tttgcttgcg acccgagtcc gctcattcgc actgaagcct gcggcagagg ccgtcgcaaa    1800
gctgactcct gagcttttgt ccgtggaggc ctggggcggc gcgacctacg atgtggcgat    1860
gcgtttcctc tttgaggatc cgtgggacag gctcgacgag ctgcgcgagg cgatgccgaa    1920
tgtaaacatt cagatgctgc ttcgcggccg caacaccgtg ggatacaccc cgtacccaga    1980
ctccgtctgc cgcgcgtttg ttaaggaagc tgccagctcc ggcgtggaca tcttccgcat    2040
cttcgacgcg cttaacgacg tctcccagat gcgtccagca atcgacgcag tcctggagac    2100
caacaccgcg gtagccgagg tggctatggc ttattctggt gatctctctg atccaaatga    2160
aaagctctac accctggatt actacctaaa gatggcagag gagatcgtca agtctggcgc    2220
tcacatcttg gccattaagg atatggctgg tctgcttcgc ccagctgcgg taaccaagct    2280

<210>  9
<211>  28
<212>  DNA
<213>  Artificial Sequence

<220>
<221>  prim_bind
<222>  (1)..(28)
<223>  pyc-P458S upstream forward primer

<400>  9
cccaagcttt gactgctcac tgcagcgt                                          28

<210>  10
<211>  17
<212>  DNA
<213>  Artificial Sequence

<220>
<221>  prim_bind
<222>  (1)..(17)
<223>  pyc-P458S mutation site reverse primer


<400>  10
aggtgcgagt gatcggc                                                      17

<210>  11
<211>  17
<212>  DNA
<213>  Artificial Sequence

<220>
<221>  prim_bind
<222>  (1)..(17)
<223>  pyc-P458S mutation site forward primer


<400>  11
gccgatcact cgcacct                                                      17

<210>  12
<211>  26
<212>  DNA
<213>  Artificial Sequence

<220>
<221>  prim_bind
<222>  (1)..(26)
<223>  pyc-P458S downstream reverse primer


<400>  12
gctctagagc gtcgattgct ggacgc                                            26

<210>  13
<211>  19
<212>  DNA
<213>  Artificial Sequence

<220>
<221>  prim_bind
<222>  (1)..(19)
<223>  pyc-P458S forward identified primer


<400>  13
cgcaaattag caacagaag                                                    19

<210>  14
<211>  18
<212>  DNA
<213>  Artificial Sequence

<220>
<221>  prim_bind
<222>  (1)..(18)
<223>  pyc-P458S reverse identified primer


<400>  14
ccttaatggc caagatgt                                                    18

<210>  15
<211>  750
<212>  DNA
<213>  Corynebacterium glutamicum

<400>  15
acctggccct cgatacctcg agtctggtgg cggctttgtc tgaagatatt tctggcgccg      60
gattaaatga cctgaaagtt ctcgacgtcg gcggcggacc cggatacttc gccgaagcct     120
ttgagacact gggcgccacc tacttctccg tcgaacccga cgttggcgaa atgtccgcag     180
ctggcatcga cgtccacgga tcagtccgcg gatccggcct cgacctgccg tttcttcccg     240
attcctttga cgtggtgtac tcctccaacg ttgcagaaca tgtctccgca ccgtgggaat     300
tgggagaaga aatgctccgc gtcacccgca gcggcggcct ggcaatcctg agctacacca     360
tttggttagg gcccttcggc ggccatgaaa ccggactgtg ggaacactac gttggcggag     420
aatttgcccg cgatcgctac acgaagaaac acgggcaccc gcctaagaac gttttcgggg     480
agtcactgtt taatgtgtcc tgccgggagg ggctggaatg gggagcctcc gtgggcaatg     540
cggaattggt tgccgctttt ccccgctacc acccgtattg ggtctggtgg atggttaaag     600
tcccagtgct ccgagaattc gcggtaagta acttggtgtt ggtgtttaaa aagcactgag     660
gttttgagga attcatcgct taacgacaag aaaggctccc actttcggtg ggagcctttc     720
ttgttattta gcagttctta agcgtgaact                                      750

<210>  16
<211>  767
<212>  DNA
<213>  Corynebacterium glutamicum

<400>  16
tacttctcca gattttgtgt cattcgacag agttctcgcc ccctagcgta gctttcagat      60
acagaactag ttaaaacttt aggtgagaca acggacacat ttgtcattac cagtggacct     120
accccctgcc cacacgcatc tacacacttt ctttaatatg agagcacccg tttaaatagc     180
ctattttggg ggtggtttca agaattaacc tcaaccgttc tccgacagtt cattccccgt     240
ccatggccat tgggttcaga tttgggcaat tctcacacat tccaggggac aactttccca     300
gttttcccac cattaacact taacattcgg acaataggca acaaaacgcc aagaacagcg     360
gtaatggtaa tccctttccc ttaccctgcc atcacaatcc aagcactccg ctagtggccg     420
accagcacaa accggcccac tgtcagtaca caccttttta aaacaacatt tacactcaca     480
tgcatgcccg cactgtcacc acccgccctc aactaccgaa ctaaagatat gtacttgaag     540
ccaaattttt accctagatc ccccttttaa atactttgaa aattactcac acacatcccc     600
acgttacccc aaaggttata tccagttagt cgtatcaaaa agtgctctga tcttaacttt     660
gccctaccta aatacatgac cccaccacga cggccagtac taacgacaga atccactagc     720
gaacccattt attaacaaac attgcaaaca agtgttgaag tattcgc                   767

<210>  17
<211>  960
<212>  DNA
<213>  Corynebacterium glutamicum

<400>  17
gctcctttta aaaaattcca cccgctgctg aaatgagcct ttacaggcct aggtaatgct      60
caagtcctac gactaggcct gggtgctgtg caatgttgcg cacacccacc aagacacctg     120
gtgcaaatga gttgcgatca taggagtcct gcttgatggt caaggtctga ccctgggtgc     180
caaagataac ttgctcgtga gcaaccatgc cggacatgcg gactgcatga accgggattc     240
catctacgct tgcgccacgg gaaccctcaa gtgcctgctc ggtcgcatct ggctgtgcgt     300
ccatgcctgc ttctttgcgt gccgcagcaa tgccctgagc agtgtggatc gcggtgcctg     360
aaggtgcatc cagcttgttg gggtggtgca gctcaataac ttcagctgat tcgaagaagc     420

gggcagcctg cttggaaaag accatggtca acaccgcaga gatagcaaag ttaggtgcga 480
tcagaacacc gacattgtct tttccttcaa gccagtcgcg aacctgctcc aaacgagcat 540
catcgaagcc cgtggttcca acaaccgcag aaatgccgtt gttgatgcag aactccaggt 600
tgcccatcac agcgttagga gtggtgaagt caacgacaac ttcagcgccg ttgtctacca 660
gaaggctcaa atcatcgtcg acgccgatct ctgcaacaag ctccagatcg tcggactcat 720
tgactgctgc cacaatagtt tgaccaacac ggcctttggc tccgagaacg ccaaccttga 780
ttcccattat gctccttcat tttcgtgggg cgaagagttt ttcaaacgcc caccagtcta 840
gacgtacccg ttcagaaggt gctgatatcc atacctaact gaccgttttg gtctgtgttg 900
ttaacgattg ttcatcagtt tgttccgttc tagggattaa tcacacaggt ggaactatgt 960

<210> 18
<211> 49
<212> DNA
<213> Artificial Sequence

<220>
<221> prim_bind
<222> (1)..(49)
<223> pck upstream homologous arms forward primer


<400> 18
tctagagtcg acctgcaggc atgcaagctt acctggccct cgatacctc 49

<210> 19
<211> 41
<212> DNA
<213> Artificial Sequence

<220>
<221> prim_bind
<222> (1)..(41)
<223> pck upstream homologous arms reverse primer


<400> 19
cctaggcctg taaagttcac gcttaagaac tgctaaataa c 41

<210> 20
<211> 40
<212> DNA
<213> Artificial Sequence

<220>
<221> prim_bind
<222> (1)..(40)
<223> pyc promoter forward primer


<400> 20
tgtgagtcga cattagagta attattcctt tcaacaagag 40

<210> 21
<211> 34
<212> DNA
<213> Artificial Sequence

<220>
<221> prim_bind
<222> (1)..(34)
<223> pyc promoter reverse primer

<400>  21
atctggagaa gtatgcgtta aacttggcca aatg                                    34

<210>  22
<211>  32
<212>  DNA
<213> Artificial Sequence

<220>
<221>  prim_bind
<222>  (1)..(32)
<223>  pyc* forward primer


<400>  22
tccgttctag ggattaggaa acgacgacga tc                                      32

<210>  23
<211>  36
<212>  DNA
<213>  Artificial Sequence

<220>
<221>  prim_bind
<222>  (1)..(36)
<223>  pyc* reverse primer


<400>  23
aggaataatt actctaatgt cgactcacac atcttc                                  36

<210>  24
<211>  31
<212>  DNA
<213>  Artificial Sequence

<220>
<221>  prim_bind
<222>  (1)..(31)
<223>  dapB forward primer


<400>  24
ttaagcgtga actttacagg cctaggtaat g                                       31

<210>  25
<211>  36
<212>  DNA
<213>  Artificial Sequence

<220>
<221>  prim_bind
<222>  (1)..(36)
<223>  dapB reverse primer


<400>  25
tcgtcgtcgt ttcctaatcc ctagaacgga acaaac                                  36

<210>  26
<211>  32
<212>  DNA
<213>  Artificial Sequence

<220>
<221>  prim_bind
<222>  (1)..(32)
<223>  pck downstream homologous arms forward primer

<400>  26
caagtttaac gcatacttct ccagattttg tg                                    32

<210>  27
<211>  49
<212>  DNA
<213>  Artificial Sequence

<220>
<221>  prim_bind
<222>  (1)..(49)
<223>  pck downstream homologous arms reverse primer

<400>  27
cgttgtaaaa cgacggccag tgccaagctt gcgaatactt caacacttg                  49

<210>  28
<211>  20
<212>  DNA
<213>  Artificial Sequence

<220>
<221>  prim_bind
<222>  (1)..(20)
<223>  pyc*-dapB identified forward primer

<400>  28
taccttgggc aggtcgtggg                                                  20

<210>  29
<211>  20
<212>  DNA
<213>  Artificial Sequence

<220>
<221>  prim_bind
<222>  (1)..(20)
<223>  pyc*-dapB identified reverse primer

<400>  29
tgggagcgtt gtgcgctcga                                                  20

<210>  30
<211>  1266
<212>  DNA
<213>  Corynebacterium glutamicum

<400>  30
atggccctgg tcgtacagaa atatggcggt tcctcgcttg agagtgcgga acgcattaga      60
aacgtcgctg aacggatcgt tgccaccaag aaggctggaa atgatgtcgt ggttgtctgc     120
tccgcaatgg gagacaccac ggatgaactt ctagaacttg cagcggcagt gaatcccgtt     180
ccgccagctc gtgaaatgga tatgctcctg actgctggtg agcgtatttc taacgctctc     240
gtcgccatgg ctattgagtc ccttggcgca gaagcccaat ctttcacggg ctctcaggct     300

ggtgtgctca ccaccgagcg ccacggaaac gcacgcattg ttgatgtcac tccaggtcgt 360
gtgcgtgaag cactcgatga gggcaagatc tgcattgttg ctggtttcca gggtgttaat 420
aaagaaaccc gcgatgtcac cacgttgggt cgtggtggtt ctgacaccac tgcagttgcg 480
ttggcagctg ctttgaacgc tgatgtgtgt gagatttact cggacgttga cggtgtgtat 540
accgctgacc cgcgcatcgt tcctaatgca cagaagctgg aaaagctcag cttcgaagaa 600
atgctggaac ttgctgctgt tggctccaag attttggtgc tgcgcagtgt tgaatacgct 660
cgtgcattca atgtgccact tcgcgtacgc tcgtcttata gtaatgatcc cggcactttg 720
attgccggct ctatggagga tattcctgtg gaagaagcag tccttaccgg tgtcgcaacc 780
gacaagtccg aagccaaagt aaccgttctg ggtatttccg ataagccagg cgaggctgcg 840
aaggttttcc gtgcgttggc tgatgcagaa atcaacattg acatggttct gcagaacgtc 900
tcttctgtag aagacggcac caccgacatc atcttcacct gccctcgttc cgacggccgc 960
cgcgcgatgg agatcttgaa gaagcttcag gttcagggca actggaccaa tgtgctttac 1020
gacgaccagg tcggcaaagt ctccctcgtg ggtgctggca tgaagtctca cccaggtgtt 1080
accgcagagt tcatggaagc tctgcgcgat gtcaacgtga acatcgaatt gatttccacc 1140
tctgagattc gtatttccgt gctgatccgt gaagatgatc tggatgctgc tgcacgtgca 1200
ttgcatgagc agttccagct gggcggcgaa gacgaagccg tcgtttatgc aggcaccgga 1260
cgctaa 1266

<210> 31
<211> 963
<212> DNA
<213> Corynebacterium glutamicum

<400> 31
atgaccaaca tccgcgtagc tatcgtgggc tacggaaacc tgggacgcag cgtcgaaaag 60
cttattgcca agcagcccga catggacctt gtaggaatct tctcgcgccg ggccaccctc 120
gacacaaaga cgccagtctt tgatgtcgcc gacgtggaca agcacgccga cgacgtggac 180
gtgctgttcc tgtgcatggg ctccgccacc gacatccctg agcaggcacc aaagttcgcg 240
cagttcgcct gcaccgtaga cacctacgac aaccaccgcg acatcccacg ccaccgccag 300
gtcatgaacg aagccgccac cgcagccggc aacgttgcac tggtctctac cggctgggat 360
ccaggaatgt tctccatcaa ccgcgtctac gcagcggcag tcttagccga gcaccagcag 420
cacaccttct ggggcccagg tttgtcacag ggccactccg atgctttgcg acgcatccct 480
ggcgttcaaa aggcagtcca gtacaccctc ccatccgaag acgccctgga aaaggcccgc 540
cgcggcgaag ccggcgacct taccggaaag caaacccaca agcgccaatg cttcgtggtt 600
gccgacgcgg ccgatcacga gcgcatcgaa aacgacatcc gcaccatgcc tgattacttc 660
gttggctacg aagtcgaagt caacttcatc gacgaagcaa ccttcgactc cgagcacacc 720
ggcatgccac acggtggcca cgtgattacc accggcgaca ccggtggctt caaccacacc 780
gtggaataca tcctcaagct ggaccgaaac ccagatttca ccgcttcctc acagatcgct 840
ttcggtcgcg cagctcaccg catgaagcag cagggccaaa gcggagcttt caccgtcctc 900
gaagttgctc catacctgct ctccccagag aacttggacg atctgatcgc acgcgacgtc 960
taa 963

<210> 32
<211> 1338
<212> DNA
<213> Corynebacterium glutamicum

<400> 32
atggctacag ttgaaaattt caatgaactt cccgcacacg tatggccacg caatgccgtg 60
cgccaagaag acggcgttgt caccgtcgct ggtgtgcctc tgcctgacct cgctgaagaa 120
tacggaaccc cactgttcgt agtcgacgag gacgatttcc gttcccgctg tcgcgacatg 180
gctaccgcat tcggtggacc aggcaatgtg cactacgcat ctaaagcgtt cctgaccaag 240
accattgcac gttgggttga tgaagagggg ctggcactgg acattgcatc catcaacgaa 300
ctgggcattg ccctggccgc tggtttcccc gccagccgta tcaccgcgca cggcaacaac 360
aaaggcgtag agttcctgcg cgcgttggtt caaaacggtg tgggacacgt ggtgctggac 420
tccgcacagg aactagaact gttggattac gttgccgctg gtgaaggcaa gattcaggac 480
gtgttgatcc gcgtaaagcc aggcatcgaa gcacacaccc acgagttcat cgccactagc 540
cacgaagacc agaagttcgg attctccctg gcatccggtt ccgcattcga agcagcaaaa 600
gccgccaaca acgcagaaaa cctgaacctg gttggcctgc actgccacgt tggttcccag 660
gtgttcgacg ccgaaggctt caagctggca gcagaacgcg tgttgggcct gtactcacag 720
atccacagcg aactgggcgt tgcccttcct gaactggatc tcggtggcgg atacggcatt 780
gcctataccg cagctgaaga accactcaac gtcgcagaag ttgcctccga cctgctcacc 840
gcagtcggaa aaatggcagc ggaactaggc atcgacgcac caaccgtgct tgttgagccc 900

ggccgcgcta tcgcaggccc ctccaccgtg accatctacg aagtcggcac caccaaagac 960
gtccacgtag acgacgacaa aacccgccgt tacatcgccg tggacggagg catgtccgac 1020
aacatccgcc cagcactcta cggctccgaa tacgacgccc gcgtagtatc ccgcttcgcc 1080
gaaggagacc cagtaagcac ccgcatcgtg ggctcccact gcgaatccgg cgatatcctg 1140
atcaacgatg aaatctaccc atctgacatc accagcggcg acttccttgc actcgcagcc 1200
accggcgcat actgctacgc catgagctcc cgctacaacg ccttcacacg gcccgccgtc 1260
gtgtccgtcc gcgctggcag ctcccgcctc atgctgcgcc gcgaaacgct cgacgacatc 1320
ctctcactag aggcataa 1338

<210> 33
<211> 58
<212> DNA
<213> Artificial Sequence

<220>
<221> prim_bind
<222> (1)..(58)
<223> lysC forward primer

<400> 33
caggtcgact ctagaggatc cccgggaaag gaggacaacc atggccctgg tcgtacag 58

<210> 34
<211> 53
<212> DNA
<213> Artificial Sequence

<220>
<221> prim_bind
<222> (1)..(53)
<223> lysC reverse primer

<400> 34
caccgacatc atcttcacct gcgttgtcct cctttttagc gtccggtgcc tgc 53

<210> 35
<211> 43
<212> DNA
<213> Artificial Sequence

<220>
<221> prim_bind
<222> (1)..(43)
<223> ddh forward primer

<400> 35
caccggacgc taaaaaggag gacaaccatg accaacatcc gcg 43

<210> 36
<211> 32
<212> DNA
<213> Artificial Sequence

<220>
<221> prim_bind
<222> (1)..(32)
<223> ddh reverse primer

<400> 36

gttgtcctcc tttttagacg tcgcgtgcga tc                                    32

<210>  37
<211>  45
<212>  DNA
<213>  Artificial Sequence

<220>
<221>  prim_bind
<222>  (1)..(45)
<223>  lysA forward primer


<400>  37
acgcgacgtc taaaaaggag gacaaccatg gctacagttg aaaat                       45

<210>  38
<211>  49
<212>  DNA
<213>  Artificial Sequence

<220>
<221>  prim_bind
<222>  (1)..(49)
<223>  lysA reverse primer


<400>  38
ctcatccgcc aaaacagcca agctgaattc ttatgcctct agtgagagg                   49

<210>  39
<211>  978
<212>  DNA
<213>  Corynebacterium glutamicum

<400>  39
atgtcgaagc agcactccac accattaaac aatgatgaag aacacacttc cgctcctcaa      60
aaggttgcgg taatcaccac gggcggaacc atcgcctgta cttccgacgc aaatgggcat     120
ctgcttccca ccgtcagcgg tgcagacctg cttgcgccaa tcgcaccacg gttcaatgga     180
gcgcagatcg ctttcgaaat ccacgaaatc aaccgccttg attcctcctc catgacgttt     240
gaggatctcg attccatcat cgccacggtt cataaggtgt tggaggatcc ggatgttgtt     300
ggcgtagtag ttacccacgg caccgattcc atggaagagt ccgccatcgc cgtagacacc     360
ttccttgatg atccccgccc agtcattttc accggcgccc aaaaaccctt cgatcatccc     420
gaagccgacg gcccaaacaa ccttttcgaa gcctgcctca tcgcatccga cccctccgct     480
cgcggaattg gtgcactcat tgtcttcggt cacgccgtca tccctgctcg cggctgcgtt     540
aaatggcaca cctctgatga gctggcgttt gcaaccaacg gccctgaaga accagagcgc     600
cccgatgcgc tgcccgtagc taaattggcg gatgtctctg tcgaaatcat ccccgcatac     660
cctggtgcca ccggcgcaat ggtggaagct gccatcgctg ccggtgctca aggacttgta     720
gtggaagcaa tgggatcagg caatgttggt tcccgcatgg gtgatgccct aggtaaagca     780
cttgacgctg gaattcccgt ggtgatgagc actagggttc ctcgtggtga agtatccgga     840
gtgtatggcg gtgcaggtgg aggtgcgact ttggctgcga agggcgctgt gggatctcgc     900
tacttcagag ctggtcaggc acgtattttg ctcgcgattg ccattgcgac gggcgcacat     960
ccggtgacgc tttactaa                                                   978

<210>  40
<211>  200
<212>  DNA
<213>  Corynebacterium glutamicum

<400>  40
tggccgttac cctgcgaatg tccacagggt agctggtagt ttgaaaatca acgccgttgc      60
ccttaggatt cagtaactgg cacattttgt aatgcgctag atctgtgtgc tcagtcttcc     120
aggctgctta tcacagtgaa agcaaaacca attcgtggct gcgaaagtcg tagccaccac     180

gaagtccagg aggacataca                                                    200

<210>  41
<211>  29
<212>  DNA
<213> Artificial Sequence

<220>
<221>  prim_bind
<222>  (1)..(29)
<223>  insert Ptuf upstream homologous arms forward primer


<400>  41
ccggaattct gctcaggagc aacagtatt                                          29

<210>  42
<211>  40
<212>  DNA
<213>  Artificial Sequence

<220>
<221>  prim_bind
<222>  (1)..(40)
<223>  insert Ptuf upstream homologous arms reverse primer


<400>  42
cattcgcagg gtaacggcca gcgctctagc gtatcaacta                              40

<210>  43
<211>  40
<212>  DNA
<213>  Artificial Sequence

<220>
<221>  prim_bind
<222>  (1)..(40)
<223>  Ptuf forward primer


<400>  43
tagttgatac gctagagcgc tggccgttac cctgcgaatg                              40

<210>  44
<211>  40
<212>  DNA
<213>  Artificial Sequence

<220>
<221>  prim_bind
<222>  (1)..(40)
<223>  Ptuf reverse primer


<400>  44
gtggagtgct gcttcgacat tgtatgtcct cctggacttc                              40

<210>  45
<211>  40
<212>  DNA
<213>  Artificial Sequence

<220>
<221>  prim_bind
<222>  (1)..(40)
<223>  insert Ptuf downstream homologous arms forward primer


<400>  45
gaagtccagg aggacataca atgtcgaagc agcactccac                              40

<210>  46
<211>  29
<212>  DNA
<213>  Artificial Sequence

<220>
<221>  prim_bind
<222>  (1)..(29)
<223>  insert Ptuf downstream homologous arms reverse primer


<400>  46
tgctctagac agcgatggca gcttccacc                                          29

<210>  47
<211>  29
<212>  DNA
<213>  Artificial Sequence

<220>
<221>  prim_bind
<222>  (1)..(29)
<223>  increase ncgl2026 copy upstream homologous arms forward primer


<400>  47
tgctctagaa agggcaatga gtttgtcga                                          29

<210>  48
<211>  40
<212>  DNA
<213>  Artificial Sequence

<220>
<221>  prim_bind
<222>  (1)..(40)
<223>  increase ncgl2026 copy upstream homologous arms reverse primer


<400>  48
gtggagtgct gcttcgacat ttagttctcc aagtagagcc                              40

<210>  49
<211>  40
<212>  DNA
<213>  Artificial Sequence

<220>
<221>  prim_bind
<222>  (1)..(40)
<223>  ncgl2026 forward primer


<400>  49

ggctctactt ggagaactaa atgtcgaagc agcactccac 40

<210> 50
<211> 39
<212> DNA
<213> Artificial Sequence

<220>
<221> prim_bind
<222> (1)..(39)
<223> ncgl2026 reverse primer


<400> 50
tatcagacga gatcttggat tagtaaagcg tcaccggat 39

<210> 51
<211> 39
<212> DNA
<213> Artificial Sequence

<220>
<221> prim_bind
<222> (1)..(39)
<223> increase ncgl2026 copy downstream homologous arms forward primer


<400> 51
atccggtgac gctttactaa tccaagatct cgtctgata 39

<210> 52
<211> 29
<212> DNA
<213> Artificial Sequence

<220>
<221> prim_bind
<222> (1)..(29)
<223> increase ncgl2026 copy downstream homologous arms reverse primer


<400> 52
ctagctagcg tgtggatccg agcgcgaag 29

<210> 53
<211> 29
<212> DNA
<213> Artificial Sequence

<220>
<221> prim_bind
<222> (1)..(29)
<223> ncgl2026 knockout upstream homologous arms forward primer


<400> 53
ccggaattct gctcaggagc aacagtatt 29

<210> 54
<211> 40
<212> DNA
<213> Artificial Sequence

<220>
<221>  prim_bind
<222>  (1)..(40)
<223>  ncgl2026 knockout upstream homologous arms reverse primer


<400>  54
atgcaagacc aagggcgaaa gcgctctagc gtatcaacta                              40

<210>  55
<211>  40
<212>  DNA
<213>  Artificial Sequence

<220>
<221>  prim_bind
<222>  (1)..(40)
<223>  ncgl2026 knockout downstream homologous arms forward primer


<400>  55
tagttgatac gctagagcgc tttcgccctt ggtcttgcat                              40

<210>  56
<211>  29
<212>  DNA
<213>  Artificial Sequence

<220>
<221>  prim_bind
<222>  (1)..(29)
<223>  ncgl2026 knockout downstream homologous arms reverse primer


<400>  56
ctagctagct tatgaggtag gcgtgcaat                                          29

<210>  57
<211>  244
<212>  DNA
<213>  Corynebacterium glutamicum

<400>  57
tgcgttaaac ttggccaaat gtggcaacct ttgcaaggtg aaaaactggg gcggggttag        60
atcctggggg gtttatttca ttcactttgg cttgaagtcg tgcaggtcag gggagtgttg        120
cccgaaaaca ttgagaggaa aacaaaaacc gatgtttgat tgggggaatc gtgtggtata       180
atggtaggac gcagtgactg ctatcaccct tggcggtctc ttgttgaaag gaataattac       240
tcta                                                                     244

## Description

### Technical Field

The present invention generally relates to the field of microbial fermentation, and specifically relates to a recombinant bacterium capable of producing L-lysine, a construction method thereof, and a production method of L-lysine.

### Background

L-lysine is one of the nine essential amino acids of the human body. It has various physiological functions such as regulating the body's metabolic balance and promoting growth and development. It is widely used in the fields of food, feed and medicine. In the feed industry, lysine is the first limiting amino acid for the growth of pigs and poultry. Adding L-lysine to the feed can improve the utilization rate of amino acids and proteins in the feed, improve the nutritional potency of the feed, and promote the growth of livestock and poultry. In the food industry, L-lysine is mainly used for nutrition enhancers and deodorants. In the field of medicine, L-lysine is one of the main components of compound amino acid preparations. At present, the lysine industry is the second largest amino acid industry after glutamic acid. Therefore, the industrial production research of L-lysine is of great significance.

At present, L-lysine is mainly produced by direct fermentation of microorganisms. The fermentation performance of lysine-producing bacteria is a key factor affecting the production cost of the fermentation method.

The breeding methods of high-producing strains of lysine mainly include traditional mutagenesis and metabolic engineering transformation.

The strains obtained through mutagenesis screening will accumulate a large number of negative-effect mutations, resulting in problems such as slow growth of the strains, reduced environmental tolerance and increased nutritional requirements. These defects limit the industrial application of strains.

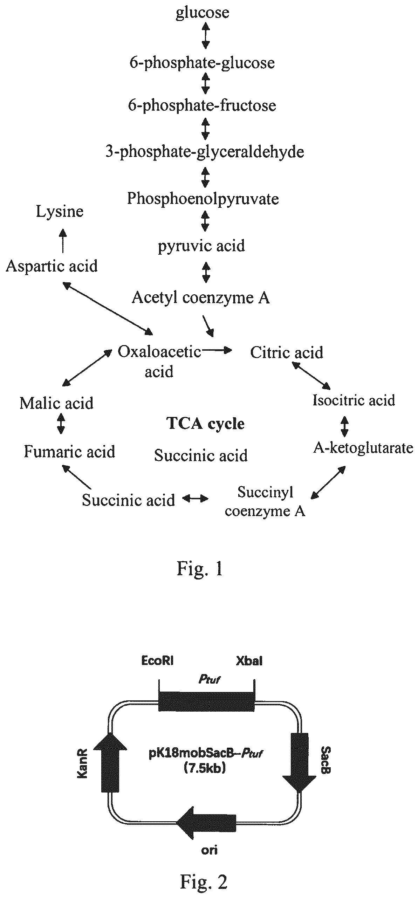

As shown in Fig. 1, in the anabolic pathway of lysine from Corynebacterium glutamicum, the synthetic precursor of lysine is oxaloacetic acid in the tricarboxylic acid cycle (TCA cycle). The oxaloacetic acid is converted into aspartic acid through transamination to enter the synthesis pathway of lysine. Therefore, the metabolic engineering transformation of lysine-producing strains in the prior art mainly focuses on the terminal synthesis pathway of lysine, the glycolysis pathway that provides synthetic precursors, the TCA cycle, and the modification of key genes in the pentose phosphate pathway that provides the cofactor NADPH. Specifically, it mainly increases the synthesis of oxaloacetate by enhancing the expression of pyruvate carboxylase gene (*pyc* gene) and weakening the expression of phosphoenolpyruvate carboxykinase gene (*pck* gene), so as to increase the accumulation of lysine. However, to date, there is no existing technology for metabolic engineering transformation of lysine-producing strains from the perspective of affecting the supply of aspartic acid.

### Summary

The inventor discovered in the previous research that the supply of aspartic acid is also a key factor affecting the synthesis of lysine. Increasing the synthesis of aspartic acid can ensure the supply of precursors for massive synthesis of lysine and increase the lysine synthesis efficiency of strains. In the metabolism process of aspartic acid, aspartic acid is catalyzed by asparagine synthase to produce asparagine, and asparagine is catalyzed by asparaginase to produce aspartic acid and ammonia.

The purpose of the present invention is to provide a recombinant bacterium capable of producing L-lysine by carrying out metabolic engineering modification of lysine-producing strains.

The present invention provides a recombinant bacterium capable of producing L-lysine, wherein the recombinant bacterium has increased expression and/or activity of asparaginase (EC 3.5.1.1 asparaginase) compared to an original bacterium. The original bacterium refers to a strain capable of accumulating lysine.

Preferably, the recombinant bacterium according to the above description, wherein the recombinant bacterium has at least two copies of asparaginase encoding gene, and/or the expression of the asparaginase encoding gene of the recombinant bacterium is mediated by a regulatory element with high transcription or high expression activity. Preferably, the regulatory element is a strong promoter. More preferably, the strong promoter is a P_{tuf} promoter of the original bacterium.

Preferably, the recombinant bacterium according to the above description, wherein the recombinant bacterium has reduced expression and/or activity of homoserine dehydrogenase (*Hom*) compared to the original bacterium. The reduced homoserine dehydrogenase expression is achieved in at least one of the following ways: (A) the homoserine dehydrogenase encoding gene of the recombinant bacterium is inactivated, and (B) the expression of the homoserine dehydrogenase encoding gene of the recombinant bacterium is mediated by a regulatory element with low transcription or low expression activity. The reduced activity of homoserine dehydrogenase is achieved by mutating the 59th valine of the homoserine dehydrogenase of the recombinant bacterium to alanine, wherein, preferably, the homoserine dehydrogenase encoding gene of the recombinant bacterium is SEQ ID NO.1.

Preferably, the recombinant bacterium according to the above description, wherein the recombinant bacterium has increased expression and/or activity of pyruvate carboxylase (*pyc*) compared to the original bacterium. Preferably, the increased expression of pyruvate carboxylase is achieved in at least one of the following ways: (C) the recombinant bacterium has at least two copies of pyruvate carboxylase encoding gene, and (D) the expression of the pyruvate carboxylase encoding gene of the recombinant bacterium is mediated by a regulatory element with high transcription or high expression activity. The increased activity of pyruvate carboxylase is achieved by mutating the 458th proline of the pyruvate carboxylase of the recombinant bacterium to serine, wherein, preferably, the pyruvate carboxylase encoding gene of the recombinant bacterium is SEQ ID NO.8.

Preferably, the recombinant bacterium according to the above description, wherein the recombinant bacterium has reduced expression and/or activity of phosphoenolpyruvate carboxykinase (*pck*) compared to the original bacterium. Preferably, the phosphoenolpyruvate carboxykinase encoding gene of the recombinant bacteria is inactivated, and/or the expression of the phosphoenolpyruvate carboxykinase encoding gene is mediated by a regulatory element with low transcription or low expression activity. More preferably, the inactivation is implemented by knocking out the phosphoenolpyruvate carboxykinase encoding gene of the recombinant bacterium.

Preferably, the recombinant bacterium according to the above description, wherein the recombinant bacterium has increased expression and/or activity of dihydropyridine dicarboxylate reductase (*dapB*) compared to the original bacterium. Preferably, the recombinant bacterium has at least two copies of dihydropyridine dicarboxylate reductase encoding gene, and/or the expression of the dihydropyridine dicarboxylate reductase encoding gene is mediated by a regulatory element with high transcription or high expression activity. More preferably, the regulatory element is a strong promoter. Most preferably, the strong promoter is a P_{tuf} promoter of the original bacterium.

Preferably, the recombinant bacterium according to the above description, wherein the recombinant bacterium has increased expression and/or activity of aspartate kinase (*lysC*), diaminopimelate dehydrogenase (*ddh*) and/or diaminopimelate decarboxylase (*lysA*) compared to the original bacterium. Preferably, the recombinant bacterium has at least two copies of aspartate kinase encoding gene, diaminopimelate dehydrogenase encoding gene and/or diaminopimelate decarboxylase encoding gene, and/or the expression of the aspartate kinase encoding gene, the diaminopimelate dehydrogenase encoding gene and/or the diaminopimelate decarboxylase encoding gene is mediated by a regulatory element with high transcription or high expression activity. More preferably, the regulatory element is a strong promoter. Most preferably, the strong promoter is a P_{tuf} promoter of the original bacterium.

Or preferably, the recombinant bacterium according to the above description, wherein the original bacterium is a bacterium selected from *Corynebacterium, Brevibacterium, Bacillus, Bifidobacterium,* and *Lactobacillus* or a fungus selected from yeast.

The bacterium of Corynebacterium is selected from *Corynebacterium glutamicum, Corynebacterium pekinense, Corynebacterium efficiens, Corynebacterium crenatum, Corynebacterium thermoaminogenes, Corynebacterium aminogenes, Corynebacterium lilium, Corynebacterium callunae,* and *Corynebacterium herculis.*

The bacterium of Brevibacterium is selected from *Brevibacteriaceae flvum, Brevibacteriaceae lactofermentum* and *Brevibacteriaceae ammoniagenes.*

The bacterium of Bacillus is selected from *Bacillus licheniformis, Bacillus subtilis or Bacillus pumilus.*

The bacterium of Bifidobacterium is selected from *Bifidobacterium bifidum, Bifidobacterium longum, Bifidobacterium breve,* and *Bifidobacterium adolescentis.*

The bacterium of Lactobacillus is selected from *Lactobacillus acidophilus, Lactobacillus casei, Lactobacillus delbrueckii subsp* and *Lactobacillus fermentum.*

The fungus of yeast is selected from *Candida utilis, Saccharomyces cerevisiae, Pichia pastoris* or *Hansenula polymorpha.*

The present invention further provides a construction method of the above-mentioned recombinant bacterium, comprising the following step: increasing the expression and/or activity of asparaginase in a original bacterium. Specifically, increasing the expression and/or activity of the asparaginase in the original bacterium is achieved by at least one of the following ways: (E) increasing the copy number of asparaginase encoding gene in the original bacterium, and (F) replacing a regulatory element for the asparaginase encoding gene in the original bacterium with a regulatory element with high transcription or high expression activity.

Preferably, the construction method further comprises the step of reducing the expression and/or activity of homoserine dehydrogenase in the original bacterium.

Preferably, the construction method further comprises the step of increasing the expression and/or activity of pyruvate carboxylase in the original bacterium.

Preferably, the construction method further comprises the step of reducing the expression and/or activity of phosphoenolpyruvate carboxykinase in the original bacterium. Specifically, reducing the expression and/or activity of phosphoenolpyruvate carboxykinase in the original bacterium is achieved by at least one of the following ways: (G) inactivating, preferably knocking out, the phosphoenolpyruvate carboxykinase encoding gene in the chromosome of the original bacterium, and (H) replacing a regulatory element for the phosphoenolpyruvate carboxykinase encoding gene in the original bacterium with a regulatory element with low transcription or low expression activity.

Preferably, the construction method further comprises the step of increasing the expression and/or activity of dihydropyridine dicarboxylate reductase in the original bacterium. Specifically, increasing the expression and/or activity of the dihydropyridine dicarboxylate reductase in the original bacterium is achieved by at least one of the following ways: (I) increasing the copy number of dihydropyridine dicarboxylate reductase encoding gene in the original bacterium, and (J) replacing a regulatory element for the dihydropyridine dicarboxylate reductase in the original bacterium with a regulatory element with high transcription or high expression activity.

Or preferably, the construction method further comprises the step of increasing the expression and/or activity of aspartate kinase, diaminopimelate dehydrogenase and/or diaminoheptanoate decarboxylase in the original bacterium. Specifically, increasing the expression and/or activity of aspartate kinase, diaminopimelate dehydrogenase and/or diaminopimelate decarboxylase in the original bacterium is achieved by at least one of the following ways: (L) increasing the copy number of aspartate kinase encoding gene, diaminopimelate dehydrogenase encoding gene and/or diaminoheptanoate decarboxylase encoding gene in the original bacterium, and (M) replacing regulatory elements for the aspartate kinase encoding gene, the diaminopimelate dehydrogenase encoding gene and/or the diaminoheptanoate decarboxylase encoding gene with regulatory elements with high transcription or high expression activity.

The present invention further provides a production method of L-lysine, including the following step: fermenting and culturing the above recombinant bacterium.

Through fermentation culture, it is observed that the recombinant bacterium capable of producing L-lysine provided by the present invention has superposition effect of increasing the production, and significantly improve the production of L-lysine. The lysine production intensity after 48h of fermentation is 0.05-5 g/L/h, and the lysine production at the end of fermentation is 1-300 g/L.

The present invention first provides a metabolic engineering strategy for increasing the supply of aspartic acid, which is a precursor of lysine synthesis, by enhancing the expression of asparaginase. It can significantly increase the production of lysine, and thus can be used in bacterial fermentation to produce lysine in practice. It has developed a new method able to increase the fermentation production of lysine. It is observed that the effect of the production increasing can be superimposed, so that it can be used in bacterial fermentation to produce lysine in practice, which is convenient for promotion and application.

### Brief Description of the Drawings

Fig. 1 is a schematic diagram of the anabolic pathway of lysine from Corynebacterium glutamicum;
Fig. 2 is a schematic diagram of recombinant plasmid YZ022;
Fig. 3 is a schematic diagram of recombinant plasmid YZ023;
Fig. 4 is a schematic diagram of recombinant plasmid YZ025;
Fig. 5 is a schematic diagram of recombinant plasmid YE019;
Fig. 6 is a schematic diagram of recombinant plasmid YZ037;
Fig. 7 is a schematic diagram of recombinant plasmid YZ039; and
Fig. 8 is a schematic diagram of recombinant plasmid YZ035.

### Detailed Description of Embodiments

The embodiments of the present invention will be described in more detail in conjunction with the accompanying drawings and embodiments, in order to provide a better understanding of the embodiments of the present invention and the advantages thereof. However, the specific embodiments and examples described below are illustrative only and should not be construed as limiting the present invention.

The present invention relates to a recombinant bacterium capable of producing L-lysine, wherein the recombinant bacterium has increased expression and/or activity of asparaginase compared to a original bacterium. The original bacterium refers to a strain capable of accumulating lysine.

Increased expression and/or activity of asparaginase can be realized based on various factors, comprising increased copy number of the coding gene, replacement of the natural promoter with a more effective strong promoter, and artificial mutations intended to increase the activity. Specifically, the gene copy number can be increased by the introduction and/or amplification of endogenous and/or exogenous alleles. As for the replacement of gene promoters, its examples comprise the introduction of endogenous and/or exogenous promoters. The promoters used have effective activity to effectively enhance the expression of downstream structural genes.

In one embodiment, the recombinant bacterium has at least two copies of asparaginase encoding gene. Specifically, the recombinant bacterium has one or more copies of endogenous and/or exogenous asparaginase encoding gene in its nuclear DNA in addition to one copy of the endogenous asparaginase encoding gene. More specifically, the nucleotide sequence of the asparaginase encoding gene can be SEQ ID NO.39.

In one embodiment, the expression of the asparaginase encoding gene of the recombinant bacterium is mediated by a regulatory element with high transcription or high expression activity. Preferably, the regulatory element is a strong promoter. More preferably, the strong promoter is a P_{tuf} promoter of the original bacterium. Specifically, at the upstream of the asparaginase encoding gene in the the nuclear DNA of the recombinant bacterium, there is an effective endogenous and/or exogenous strong promoter, resulting in an effective increase in the expression of the asparaginase encoding gene.

The "original strain" in the present invention refers to the initial strain used in the genetic modification strategy of the present invention. The strain may be a naturally occurring strain, or may be a strain bred by mutagenesis or genetic engineering.

The expression "inactivation" in the present invention refers to "inactivation" in the present invention refers to that the corresponding modified object changes to achieve a certain effect, including but not limited to, site-directed mutation, insertional inactivation and/or knockout.

The methods of gene knockout, gene insertion, promoter replacement and site-directed mutation described in the present invention can be realized by homologous recombination of a homologous arm with a modified target gene carried by a vector.

The introduction of a gene or the increase in the copy number of a gene according to the present invention can be achieved by constructing a recombinant plasmid containing the gene and then introducing the recombinant plasmid into the original bacterium, or by directly inserting a gene into a suitable site on the chromosome of the original bacterium.

Although examples of regulatory elements with high transcription or high expression activity are given in the present invention, the regulatory elements with high transcription or high expression activity are not particularly limited in the present invention, as long as they can enhance the expression of the promoter genes. The regulatory elements that can be used in the present invention comprise P₄₅, P_{eftu}, P_{sod}, P_{glyA}, P_{pck}, P_{pgk} promoters of the original bacterium, etc. but are not limited thereto. The regulatory elements with low transcription or low expression activity are also not particularly limited in the present invention, as long as they can reduce the expression of the gene to be promoted.

The experimental methods in the following embodiments are conventional methods unless otherwise specified. Unless otherwise specified, the materials and reagents used in the following embodiments can be commercially available.

Unless otherwise specified in the following embodiments, the technical means used in the embodiments are conventional means well known to those skilled in the art, see "Molecular Cloning: A Laboratory Manual (3rd Edition)" (Science Press), "Microbiology Experiment (4th Edition)" (Higher Education Press), the manufacturer's instructions for the corresponding instruments and reagents, etc. Instruments and reagents used in the embodiments are commonly used instruments and reagents in the market. For the quantitative tests in the following embodiments, three replicate experiments are set, and the results are averaged.

Example 1 construction of lysine chassis engineering bacterium

In this example, the site-directed mutation was performed on *hom* (homoserine dehydrogenase, GenBank: CAF19887.1) gene of the original strain Corynebacterium glutamicum wild-type ATCC13032 to reduce the metabolic flux of a branch pathway, i.e., the synthesis pathway of threonine; site-directed mutation was performed *on pyc* (Pyruvate carboxylase, GenBank: CAF19394.1) gene to increase the supply of oxaloacetate which is a synthesis precursor of lysine; knockout *of pck* (phosphoenolpyruvate carboxykinase, GenBank: CAF20888.1) gene was performed and the copy number *of pyc** and *dapB* (dihydropyridine dicarboxylate reductase, GenBank: CAF20314.1) gene were also increased; *lysC* (aspartate kinase, GenBank: CAF18822.1), *ddh* (Diaminopimelate dehydrogenase, GenBank: CAF21279.1), and *lysA* (diaminoheptanoate decarboxylase, GenBank: CAF19884.1) genes of plasmids were overexpressed to further enhance the synthesis pathway of lysine to construct a lysine-producing chassis engineering bacterium.

### (1) Site-directed mutation of chromosome hom gene

Primers were designed respectively according to the *hom* gene of Corynebacterium glutamicum ATCC13032 in Genbank and its upstream and downstream sequences.

Using the genomic DNA of Corynebacterium glutamicum ATCC13032 as a template, primers were designed at the mutation site to amplify two parts of the *hom* gene at upstream and downstream of the mutation site, respectively. The upper half of the *hom* gene was amplified with P1 and P2 as primers, and the lower half of the *hom* gene was amplified with P3 and P4 as primers. Then using the above purified PCR product as a template and P1 and P4 as primers, SOE (gene splicing by overlap extension) PCR was performed for amplification, to obtain 1638bp PCR product, which contains *hom* gene (SEQ ID NO. 1) with the 59th valine mutated to alanine (V59A Mutation).

The above 1638bp PCR product was double-digested with Xba I and EcoR I, and then ligated with the double-digested homologous recombinant vector pK18mobsacB (purchased from ATCC, Cat. No. 87097). The ligation product was transformed into E. coli DH5α by chemical transformation, and the transformants were screened on LB plates containing kanamycin (50 µg/mL). After the subculture for three generations, transformants were identified by colony PCR using P5 and P6 as primers; a plasmid was extracted from the transformant identified positive, and the plasmid was double digested by Xba I and EcoR I and identified; the obtained 1638bp plasmid was positive.

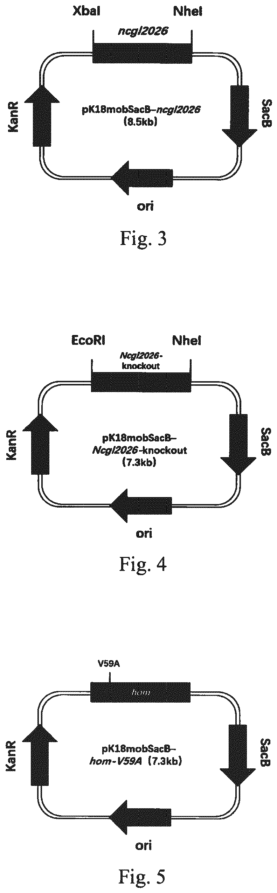

The positive plasmid was sent for sequencing. As a result, the plasmid was a recombinant plasmid obtained by inserting the nucleotide shown in SEQ ID NO. 1 in the sequence table into the vector pK18mobsacB, and named YE019, shown in fig. 5.

**Table 1**

| Primer | Base sequence | SEQ ID NO. |
|---|---|---|
| P1 | GCTCTAGAAGCTGTTTCACAATTTCT | 2 |
| P2 | ATATCAGAAGCAGCAATGC | 3 |
| P3 | GCATTGCTGCTTCTGATAT | 4 |
| P4 | CCGGAATTCCCAACAACTTGATGGTGT | 5 |
| P5 | TCTACGTTGTATCTCGCAC | 6 |
| P6 | CAGGCGACCAGCTGCTTC | 7 |

The homologous recombinant plasmid YE019 sequenced positive was electrotransformed into Corynebacterium glutamicum wild-type ATCC13032. Colonies with recombinant plasmids integrated into chromosomes were obtained by kanamycin resistance forward screening. Positive colonies with second homologous recombination were obtained by sucrose reverse screening. Using P5 and P6 as primers, PCR amplification and identification was carried out on the positive colonies to obtain a recombinant bacterium identified positive, named Corynebacterium glutamicum EPCG1000.

The genomic DNA of the recombinant bacterium was extracted and sequenced. The results confirmed that the *hom* gene in Corynebacterium glutamicum wild-type ATCC13032 had been successfully replaced with the *hom* gene at V59A, and Corynebacterium glutamicum EPCG1000 was successfully constructed.

### (2) Site-directed mutation of chromosome pyc gene

Primers were designed respectively according to the *pyc* gene of Corynebacterium glutamicum ATCC13032 in Genbank and its upstream and downstream sequences.

Using the genomic DNA of Corynebacterium glutamicum ATCC13032 as a template, primers were designed at the mutation site to amplify two parts of the *pyc* gene at upstream and downstream of the mutation site, respectively. The upper half of the *pyc* gene was amplified with P7 and P8 as primers, and the lower half of the *pyc* gene was amplified with P9 and P10 as primers. Then using the purified PCR product as a template and P7 and P10 as primers, SOE (gene splicing by overlap extension) PCR was performed for amplification, to obtain 3423bp PCR product, which was *pyc* gene (SEQ ID NO. 8) with the 458th proline mutated to alanine (P458S Mutation), i.e., *pyc** gene.

The above 3423bp PCR product was double-digested with Xba I and Hind III, and then ligated with the double-digested homologous recombinant vector pK18mobsacB (purchased from ATCC, Cat. No. 87097). The ligation product was transformed into E. coli DH5α by chemical transformation, and the transformants were screened on LB plates containing kanamycin (50 µg/mL). After the subculture for three generations, the transformants were identified by colony PCR using P11 and P12 as primers; a plasmid was extracted from the transformant identified positive, and the plasmid was double digested by Xba I and Hind III and identified; the obtained 3423bp plasmid was positive.

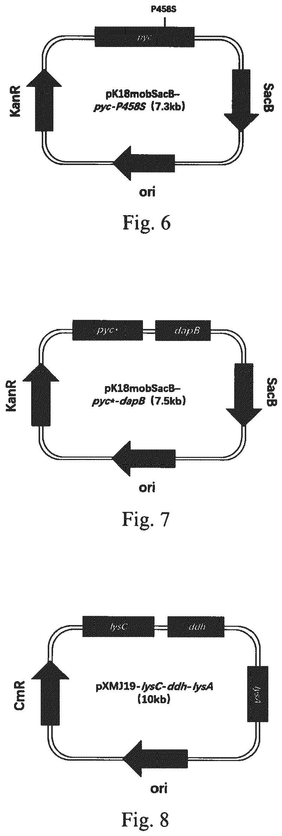

The positive plasmid was sent for sequencing. As a result, the plasmid was a recombinant plasmid obtained by inserting the nucleotide shown in SEQ ID NO. 8 in the sequence table into the vector pK18mobsacB, and named YZ037 shown in fig.6.

**Table 2**

| Primer | Base sequence | SEQ ID NO. |
|---|---|---|
| P7 | CCCAAGCTTTGACTGCTCACTGCAGCGT | 9 |
| P8 | AGGTGCGAGTGATCGGC | 10 |
| P9 | GCCGATCACTCGCACCT | 11 |
| P10 | GCTCTAGAGCGTCGATTGCTGGACGC | 12 |
| P11 | CGCAAATTAGCAACAGAAG | 13 |
| P12 | CCTTAATGGCCAAGATGT | 14 |

The homologous recombinant plasmid YZ037 sequenced positive was electrotransformed into Corynebacterium glutamicum EPCG1000. Colonies with recombinant plasmids integrated into chromosomes were obtained by kanamycin resistance forward screening. Positive colonies with second homologous recombination were obtained by sucrose reverse screening. Using P11 and P12 as primers, PCR amplification and identification was carried out on the positive colonies to obtain a recombinant bacterium identified positive, named Corynebacterium glutamicum EPCG1007.

The genomic DNA of the recombinant bacterium was extracted and sequenced. The results confirmed that the *pyc* gene in Corynebacterium glutamicum EPCG1000 had been successfully replaced with the *pyc** gene having a mutation at P458S, and Corynebacterium glutamicum EPCG1007 was successfully constructed.

### (3) Knockout of pck gene and increase in copies of pyc*-dapB

Primers were designed respectively according to the *pck* gene of Corynebacterium glutamicum ATCC13032 in Genbank and its upstream and downstream sequences.

Using the genomic DNA of Corynebacterium glutamicum ATCC13032 as a template, the sequence (SEQ ID NO.15) of the upstream part of the *pck* gene was amplified with P13 and P14 as primers, the promoter of *pyc* gene was amplified with P15 and P16 as primers, the sequence of the downstream part of the *pck* gene (SEQ ID NO. 16) was amplified with P21 And P22 as primers. Using the purified PCR products described above as the upstream and downstream homologous arms of the *pyc**-*dapB* operon, respectively, when they were integrated into the genome of Corynebacterium glutamicum ATCC13032, the purpose of knocking out *pck* could be achieved.

Using the *pyc** gene with point mutation constructed in (2) and the genomic DNA of Corynebacterium glutamicum ATCC13032 as templates, primers were designed to amplify *pyc** and *dapB* (SEQ ID NO. 17), respectively. The base information of related gene sequences was obtained from the NCBI database, and totally six pairs of primers were designed to construct the *pyc**-*dapB* gene fragment (Table 3).

**Table 3**

| Primer | Base sequence | SEQ ID NO. |
|---|---|---|
| P13 | tctagagtcgacctgcaggcatgcaagctt ACCT GGCCCT CGATACCT C | 18 |
| P14 | cctaggcctgtaaAGTTCACGCTTAAGAACTGCTAAATAAC | 19 |
| P15 | tgtgagtcgacatTAGAGTAATTATTCCTTTCAACAAGAG | 20 |
| P16 | atctggagaagtaTGCGTTAAACTTGGCCAAATG | 21 |
| P17 | tccgttctagggaTTAGGAAACGACGACGATC | 22 |
| P18 | aggaataattactctaAT GT CGACT C AC AC AT CTTC | 23 |
| P19 | ttaagcgtgaactTTACAGGCCTAGGTAATG | 24 |
| P20 | tcgtcgtcgtttcctaaTCCCTAGAACGGAACAAAC | 25 |
| P21 | caagtttaacgcaTACTTCTCCAGATTTTGTG | 26 |
| P22 | cgttgtaaaacgacggccagtgccaagcttGCGAATACTTCAACACTTG | 27 |
| P23 | taccttgggcaggtcgtggg | 28 |
| P24 | tgggagcgttgtgcgctcga | 29 |

Using the genomic DNA of Corynebacterium glutamicum ATCC13032 as a template, the genes with lengths of 750bp, 244bp, 3423bp, 896bp and 767bp were amplified with P13 and P14, P15 and P16, P17 and P18, P19 and P20, P21 and P22, respectively. These genes were the sequence of the upstream part of the *pck* gene, the promoter sequence of the *pyc* gene (SEQ ID NO. 57), the sequence of the *pyc** gene, the sequence of the *dapB* gene, and the sequence of the downstream part of the *pck* gene.

The purified PCR product was mixed with an E. coli cloning vector pK18mobsacB, ligated and assembled using NEbuilder (NEBuilder HiFi DNA Assembly Cloning Kit), and then transformed into E. coli DH5α; transformants were screened on LB plates containing kanamycin (50 µg/mL). After the subculture for three generations, the transformants were identified by colony PCR using P23 and P24 as primers; a plasmid was extracted from the transformant identified positive.

The positive plasmid was sent for sequencing. As a result, the plasmid was a recombinant plasmid obtained by inserting *pyc**-*dapB* into the vector pK18mobsacB, and named YZ039, shown in Fig. 7.

The homologous recombinant plasmid YZ039 sequenced positive was electrotransformed into Corynebacterium glutamicum EPCG1007. Colonies with recombinant plasmids integrated into chromosomes were obtained by kanamycin resistance forward screening. Positive colonies with second homologous recombination were obtained by sucrose reverse screening. Using P23 and P24 as primers, PCR amplification and identification was carried out on the positive colonies to obtain a recombinant bacterium identified positive, named Corynebacterium glutamicum EPCG1009.

The genomic DNA of the recombinant bacterium was extracted and sequenced. The results confirmed that the *pck* gene in Corynebacterium glutamicum EPCG1007 had been successfully knocked out, the *pyc**-*dapB* gene segment was also inserted, and Corynebacterium glutamicum EPCG1009 was successfully constructed.

### (4) Increase in copies of lysC, ddh and lysA genes

Primers were designed respectively according to the *lysC* (SEQ ID NO.30), *ddh* (SEQ ID NO.31), and *lysA* (SEQ ID NO.32) genes of Corynebacterium glutamicum ATCC13032 in Genbank and their upstream and downstream sequences.

Using the genomic DNA of Corynebacterium glutamicum ATCC13032 as a template, primers were designed to amplify *lysC, ddh,* and *lysA* genes respectively.

**Table 4**

| Primer | Base sequence | SEQ ID NO. |
|---|---|---|
| P25 | caggtcgactctagaggatccccggg AAAGGAGGACAACCATGGCcctggtcgtacag | 33 |
| P26 | CACCGACATCATCTTCACCTGC gttgtcctcctttTTAGCGTCCGGTGCCTGC | 34 |
| P27 | caccggacgctaaAAAGGAGGACAAC CATGACCAACATCCGCG | 35 |
| P28 | gttgtcctcctttTTAGACGTCGCGTGCGATC | 36 |
| P29 | acgcgacgtctaaAAAGGAGGACA ACCATGGCTACAGTTGAAAAT | 37 |
| P30 | ctcatccgccaaaacagccaagctgaattc TTATGCCTCTAGTGAGAGG | 38 |

Using the genomic DNA of Corynebacterium glutamicum ATCC13032 as a template, the genes with lengths of 1266bp, 963bp and 1338bp were amplified with P25 and P26, P27 and P28, P29 and P30.

The purified PCR product was mixed with an E. coli cloning vector pXMJ19, ligated and assembled using NEbuilder (NEBuilder HiFi DNA Assembly Cloning Kit), and then transformed into E. coli DH5α; transformants were screened on LB plates containing chloromycetin (20 µg/mL). After the subculture for three generations, the transformants were identified by colony PCR using P25 and P30 as primers; a plasmid was extracted from the transformant identified positive.

The positive plasmid was sent for sequencing. As a result, the plasmid was a recombinant plasmid obtained by inserting *lysC, ddh,* and *lysA* into the vector pXMJ19, and named YZ035, shown in Fig. 8.

The homologous recombinant plasmid YZ035 sequenced positive was electrotransformed into Corynebacterium glutamicum EPCG1009. The positive colonies that can grow on the resistant plate were identified by PCR amplification using P25 and P30 as primers to obtain the recombinant bacterium identified positive, named Corynebacterium glutamicum EPCG1010.

The genomic DNA of the recombinant bacterium was extracted and sequenced. The results confirmed that the free plasmid YZ035 was successfully introduced into Corynebacterium glutamicum EPCG1009, and Corynebacterium glutamicum EPCG1010 was successfully constructed.

Example 2 Promoter replacement of asparaginase encoding gene *NCgl2026* in lysine chassis engineering bacterium

Primers were designed respectively according to the upstream and downstream sequences of the *NCgl2026* gene promoter and P_{tuf} promoter sequence (SEQ ID NO. 40) of Corynebacterium glutamicum ATCC13032 in Genbank.

Using the genomic DNA of Corynebacterium glutamicum ATCC13032 as a template, the upstream homologous arm of the *NCgl2026* gene promoter was amplified by PCR with P31 and P32 as primers; the promoter P_{tuf} was amplified with P33 and P34 as primers; and the downstream homologous arm of the *NCgl2026* gene promoter was amplified with P35 and P36 as primers. Using the purified PCR product as a template and P31 and P36 as primers, SOE PCR was performed for amplification to obtain a 1800bp PCR product, which is a segment containing upstream and downstream homologous arms of the replacement promoter P_{tuf} and the replaced promoter P_{tuf}.

The above 1800bp PCR product was double-digested with Xba I and EcoR I, and then ligated with the double-digested homologous recombinant vector pK18mobsacB (purchased from ATCC, Cat. No. 87097). The ligation product was transformed into E. coli DH5α by chemical transformation, and the transformants were screened on LB plates containing kanamycin (50 µg/mL). After the subculture for three generations, the transformants were identified by colony PCR using P31 and P36 as primers to obtain a 1800bp positive transformant; the plasmid was extracted from the transformant identified positive, and the plasmid was double digested by Xba I and EcoR I and identified; the obtained 1800bp plasmid was positive.

The positive plasmid was sent for sequencing. As a result, the plasmid was a recombinant plasmid (shown in Fig. 2) obtained by inserting the strong promoter P_{tuf} containing upstream and downstream homologous arms into the vector pK18mobsacB, and named YZ022, shown in Fig.2.

**Table 6**

| Primer | Base sequence | SEQ ID NO. |
|---|---|---|
| P31 | CCGGAATTCTGCTCAGGAGCAACAGTATT | 41 |
| P32 | CATTCGCAGGGTAACGGCCAGCGCTCTAGCGTATCAACTA | 42 |
| P33 | TAGTTGATACGCTAGAGCGCTGGCCGTTACCCTGCGAATG | 43 |
| P34 | GTGGAGTGCTGCTTCGACATTGTATGTCCTCCTGGACTTC | 44 |
| P35 | GAAGTCCAGGAGGACATACAATGTCGAAGCAGCACTCCAC | 45 |
| P36 | TGCTCTAGACAGCGATGGCAGCTTCCACC | 46 |

The homologous recombinant plasmid YZ022 sequenced positive was electrotransformed into Corynebacterium glutamicum EPCG1010. Colonies with recombinant plasmids integrated into chromosomes were obtained by kanamycin resistance forward screening. Positive colonies with second homologous recombination were obtained by sucrose reverse screening. Using P31 and P36 as primers, PCR amplification and identification was carried out on the positive colonies to obtain a 1800bp recombinant bacterium, named Corynebacterium glutamicum EPCG1036.

The genomic DNA of the recombinant bacterium was extracted and sequenced. The results confirmed that the *NCgl2026* promoter in Corynebacterium glutamicum EPCG1010 was successfully replaced with the endogenous strong promoter P_{tuf} of Corynebacterium glutamicum, and Corynebacterium glutamicum EPCG1036 was successfully constructed.

Example 3 Increase of copies of asparaginase encoding gene *NCgl2026* in lysine chassis engineering bacterium

Primers were designed according to the *NCgl2026* gene of Corynebacterium glutamicum ATCC13032 in Genbank and its upstream and downstream sequences.

Using Corynebacterium glutamicum ATCC13032 genomic DNA as a template, the upstream sequence of the target insertion site was amplified by PCR with P37 and P38 as primers to function as the upstream homologous arm for the increase of copies of the *NCgl2026* gene; the *NCgl2026* gene was amplified with P39 and P40 as primers; the downstream sequence of the target insertion site was amplified with P41 and P42 as primers to function as the downstream homologous arm for the increase of copies of the *NCgl2026* gene. Using the purified PCR product as a template and P37 and P42 as primers, SOE PCR was performed for amplification to obtain a 2778bp PCR product, which is a segment containing the upstream and downstream homologous arms of the target insertion site and the *NCgl2026* gene.

The above 2778bp PCR product was double-digested with Xba I and Nhe I, and then ligated with the double-digested homologous recombinant vector pK18mobsacB (purchased from ATCC, Cat. No. 87097). The ligation product was transformed into E. coli DH5α by chemical transformation, and the transformants were screened on LB plates containing kanamycin (50 µg/mL). After the subculture for three generations, transformants were identified by colony PCR using P37 and P42 as primers to obtain a 2778bp positive transformant; a plasmid was extracted from the transformant identified positive, and the plasmid was double digested by Xba I and Nhe I and identified; the obtained 2778bp plasmid was positive.

The positive plasmid was sent for sequencing. As a result, the plasmid was a recombinant plasmid (shown in Fig. 3) obtained by inserting the upstream and downstream homologous arms of the target insertion site and the *NCgl2026* gene into the vector pK18mobsacB, and named YZ023, shown in fig.3.

**Table 7**

| Primer | Base sequence | SEQ ID NO. |
|---|---|---|
| P37 | TGCTCTAGAAAGGGCAATGAGTTTGTCGA | 47 |
| P38 | GTGGAGTGCTGCTTCGACATTTAGTTCTCCAAGTAGAGCC | 48 |
| P39 | GGCTCTACTTGGAGAACTAAATGTCGAAGCAGCACTCCAC | 49 |
| P40 | TATCAGACGAGATCTTGGATTAGTAAAGCGTCACCGGAT | 50 |
| P41 | ATCCGGTGACGCTTTACTAATCCAAGATCTCGTCTGATA | 51 |
| P42 | CTAGCTAGCGTGTGGATCCGAGCGCGAAG | 52 |

The homologous recombinant plasmid YZ023 sequenced positive was electrotransformed into Corynebacterium glutamicum EPCG1010. Colonies with recombinant plasmids integrated into chromosomes were obtained by kanamycin resistance forward screening. Positive colonies with second homologous recombination were obtained by sucrose reverse screening. Using P37 and P42 as primers, PCR amplification and identification was carried out on the positive colonies to obtain a 2778bp recombinant bacterium, named Corynebacterium glutamicum EPCG1039.

The genomic DNA of the recombinant bacterium was extracted and sequenced. The results confirmed that a copy of the *NCgl2026* gene has been successfully inserted at the target site in Corynebacterium glutamicum EPCG1010, and Corynebacterium glutamicum EPCG1039 has been successfully constructed.

Example 4 Knockout of asparaginase encoding gene *NCgl2026* from lysine chassis engineering bacterium

Primers were designed according to the *NCgl2026* gene of Corynebacterium glutamicum ATCC13032 in Genbank and its upstream and downstream sequences.

Using the genomic DNA of Corynebacterium glutamicum ATCC 13032 as a template, the upstream homologous arm of the *NCgl2026* gene was amplified by PCR with P43 and P44 as primers; and the downstream homologous arm of the *NCgl2026* gene was amplified with P45 and P46 as primers. Using the purified PCR product as a template and P43 and P46 as primers, SOE PCR was performed for amplification to obtain a 1600bp PCR product, which is a segment containing the upstream and downstream homologous arms of the *NCgl2026* gene.

The above 1600bp PCR product was double-digested with EcoR I and Nhe I, and then ligated with the double-digested homologous recombinant vector pK18mobsacB (purchased from ATCC, Cat. No. 87097). The ligation product was transformed into E. coli DH5α by chemical transformation, and the transformants were screened on LB plates containing kanamycin (50 µg/mL). After the subculture for three generations, transformants were identified by colony PCR using P43 and P46 as primers to obtain a 1600bp positive transformant; a plasmid was extracted from the transformant identified positive, and the plasmid was double digested by EcoR I and Nhe I and identified; the obtained 1600bp plasmid was positive.

The positive plasmid was sent for sequencing. As a result, the plasmid was a recombinant plasmid obtained by inserting the fragment containing the upstream and downstream homologous arms of the *NCgl2026* gene into the vector pK18mobsacB, and named YZ025, shown in Fig. 4.

**Table 8**

| Primer | Base sequence | SEQ ID NO. |
|---|---|---|
| P43 | CCGGAATTCTGCTCAGGAGCAACAGTATT | 53 |
| P44 | ATGCAAGACCAAGGGCGAAAGCGCTCTAGCGTATCAACTA | 54 |
| P45 | TAGTTGATACGCTAGAGCGCTTTCGCCCTTGGTCTTGCAT | 55 |
| P46 | CTAGCTAGCTTATGAGGTAGGCGTGCAAT | 56 |

The homologous recombinant plasmid YZ025 sequenced positive was electrotransformed into Corynebacterium glutamicum EPCG1010. Colonies with recombinant plasmids integrated into chromosomes were obtained by kanamycin resistance forward screening. Positive colonies with second homologous recombination were obtained by sucrose reverse screening. Using P43 and P46 as primers, PCR amplification and identification was carried out on the positive colonies to obtain a 1600bp recombinant bacterium, named Corynebacterium glutamicum EPCG1038.

The genomic DNA of the recombinant bacterium was extracted and sequenced. The results confirmed that the *NCgl2026* gene was knocked out from Corynebacterium glutamicum EPCG1010, and Corynebacterium glutamicum EPCG1038 was successfully constructed.

Example 5 Application of lysine engineering bacteria of Corynebacterium glutamicum in fermentation production of lysine

The L-lysine-producing Corynebacterium glutamicum EPCG1036, EPCG1038, and EPCG1039 constructed in Examples 2 to 4 and the original strain EPCG1010 were cultured at the shake flask level and the 3L fermentor level respectively to produce L-lysine as follows.

### (1) Shake flask fermentation:

Corynebacterium glutamicum EPCG1036, EPCG1038, EPCG1039 and EPCG1010 were inoculated in 500 ml Erlenmeyer flasks containing 50 ml of the seed medium described below, and cultured with shaking at 220rpm for 8-9h at 30°C. Then, 5 ml of each seed culture solution was inoculated into a 500 ml baffled bottle containing 50 ml of the fermentation medium described below, and cultured with shaking at 220rpm for 42-46h at 37°C. After fermentation for 6h, isopropyl-β-D-thiogalactopyranoside (IPTG) with a final concentration of 1 mmol/L was added to induce the expression of the target gene. Concentrated ammonia water was intermittently supplemented to control the pH of the fermentation broth between 7.0 and 7.2. According to the residual sugar, glucose mother liquor with a concentration of 400g/L was added to control the residual sugar of the fermentation broth at 5-10g/L.

### (2) 3L fermentor fermentation:

Corynebacterium glutamicum EPCG1036, EPCG1038, EPCG1039 and EPCG1010 were inoculated in 1000ml Erlenmeyer flasks containing 100ml of the seed medium described below, and cultured with shaking at 220rpm for 8-9h at 30 °C. Then, each seed culture solution was inoculated into a 3L fermentor containing 900 ml of the fermentation medium described below, and cultured under the pressure of 0.01 MPa for 42-46h at 37 °C. The seed solution was inoculated at 10vol% into a fermentation medium containing chloromycetin with a final concentration of 10 µg/ml. The fermentor used is a 3L fermentor: equipped with a built-in constant-speed programmable control pump, which can achieve constant-speed feeding. During the fermentation process, 600g/L glucose was supplemented by a peristaltic pump to control the concentration of glucose in the fermentation system at 5-10 g/L, and the fermentation temperature was maintained at 30°C by virtue of a heating jacket and cooling water; the air was supplied to provide dissolved oxygen, and the rotation speed and dissolved oxygen signal were cascaded to control the dissolved oxygen at 30%; concentrated ammonia was supplemented to adjust the pH at about 6.9. The fermentation continued for 52h. When OD₆₀₀ = 4-5, IPTG (isopropylthiogalactoside, the final concentration is 0.1mmol / L) was added to induce expression of the gene carried by the recombinant plasmid.

The seed medium and fermentation medium are as follows:

### Seed medium (pH 7.0)

20g of sucrose, lOg of peptone, 5g of yeast extract, 3.5g of urea, 4g of monopotassium phosphate, lOg of dipotassium phosphate, 0.5g of magnesium sulfate heptahydrate, 0.2mg of biotin, 1.5mg of vitamin B1, 2mg of calcium dextrose, and 3 mg of nicotinamide (dissolved in 1L of distilled water).

### Fermentation medium (pH 7.0)

40g of glucose, 20g of molasses, 0.4g of phosphoric acid, 15g of ammonium sulfate, 0.87g of magnesium sulfate heptahydrate, 0.88mg of biotin, 6.3mg of vitamin B1, 6.3mg of calcium dextropantothenate, and 42mg of nicotinamide (dissolved in 1L of distilled water).

### (3) Detection of lysine production

### HPLC method:

### 1. Mobile phase:

Organic phase: methanol: acetonitrile: water = 45:45:10 (V/V);

Aqueous phase: 12.436g of NaH₂PO₄ · 2H₂O is dissolved in 2L of ultrapure water and the pH of the obtained solution is adjusted to 7.8 with NaOH.

### 2. Elution procedure:

| Time (min) | Aqueous phase (%) | Organic phase (%) |
|---|---|---|
| 0.00 | 100.0 | 0.0 |
| 1.90 | 100.0 | 0.0 |
| 18.10 | 43.0 | 57.0 |
| 18.60 | 0.0 | 100.0 |
| 22.30 | 0.0 | 100.0 |
| 23.20 | 100.0 | 0.0 |
| 26.00 | 100.0 | 0.0 |

Solutions with standard concentration were prepared with a standard lysine product, the concentrations were 0.2g/L, 0.4g/L, 0.8g/L, 1.6g/L, and standard curves were plotted according to the peak area to calculate the concentrations of lysine in the fermentation broth as follows:

**Table 9**

| Lysine (g/L) | EPCG1036 | EPCG1038 | EPCG1039 | EPCG1010 |
|---|---|---|---|---|
| Shake flask fermentation | 10.60±0.19 | 6.24±0.11 | 12.93±0.21 | 7.46±0.35 |
| 3L Fermentor fermentation | 14.17±0.49 | 8.96±0.43 | 19.68±0.66 | 12.34±0.18 |

The results of shake flask fermentation experiments showed that the expression of the asparaginase gene was enhanced, and the production of lysine was increased significantly; after knockout of the gene, the production of lysine was decreased significantly.

Corresponding to the results of the shake flask fermentation, when fermenting at the 3L fermentor level, by increasing a copy of the asparaginase encoding gene, the production of lysine was increased by 59.48%; by replacing the asparaginase encoding gene promoter with a strong promoter, the production of lysine was increased by 14.83%; by knocking out the asparaginase encoding gene, the production of lysine was decreased by 27.39%.

Example 6 Enhanced expression of asparaginase encoding gene *NCgl2026* in Corynebacterium pekinense 1.563

Taking Corynebacterium pekinense AS 1.563 capable of accumulating lysine as the original strain, the effect of the expression of the asparaginase encoding gene on the accumulation of lysine was analyzed.

### (1) Strong promoter replacement of NCgl2026 gene

The recombinant vector YZ022 constructed in Example 2 was transformed into Corynebacterium pekinense AS 1.563 (China Center of Industrial Culture Collection, CICC10178) to achieve the replacement of the *NCgl2026* gene promoter with the strong promoter P_{tuf}.

Colonies with recombinant plasmids integrated into chromosomes were obtained by kanamycin resistance forward screening. Positive colonies with second homologous recombination were obtained by sucrose reverse screening. Using P31 and P36 as primers, PCR amplification and identification was carried out on the positive colonies to obtain a 1800bp recombinant bacterium, named Corynebacterium glutamicum CP1008.

The genomic DNA of the recombinant bacterium was extracted and sequenced. The results confirmed that the *NCgl2026* promoter in Corynebacterium pekinense AS 1.563 was successfully replaced with the endogenous strong promoter P_{tuf} of Corynebacterium glutamicum, and Corynebacterium glutamicum CP1008 was successfully constructed.

### (2) Increase of copies of NCgl2026 gene

The recombinant vector YZ023 constructed in Example 3 was transformed into Corynebacterium pekinense AS 1.563 to increase copies of the *NCgl2026* gene.

Colonies with recombinant plasmids integrated into chromosomes were obtained by kanamycin resistance forward screening. Positive colonies with second homologous recombination were obtained by sucrose reverse screening. Using P37 and P42 as primers, PCR amplification and identification was carried out on the positive colonies to obtain a 2778bp recombinant bacterium, named Corynebacterium glutamicum CP1009.

The genomic DNA of the recombinant bacterium was extracted and sequenced. The results confirmed that a copy of the *NCgl2026* gene has been successfully inserted at the target site in Corynebacterium pekinense AS 1.563, and Corynebacterium pekinense CP1009 has been successfully constructed.

(3) Knockout of asparaginase encoding gene *NCgl2026* from Corynebacterium pekinense AS 1.563

The recombinant vector YZ025 constructed in Example 4 was transformed into Corynebacterium pekinense AS 1.563 to knock out the *NCgl2026* gene.

Colonies with recombinant plasmids integrated into chromosomes were obtained by kanamycin resistance forward screening. Positive colonies with second homologous recombination were obtained by sucrose reverse screening. Using P43 and P46 as primers, PCR amplification and identification was carried out on the positive colonies to obtain a 1600bp recombinant bacterium, named Corynebacterium glutamicum CP1010.

The genomic DNA of the recombinant bacterium was extracted and sequenced. The results confirmed that the *NCgl2026* gene was knocked out from Corynebacterium pekinense AS1.563, and Corynebacterium pekinense CP1010 was successfully constructed.

Example 7 Application of lysine engineering bacteria of Corynebacterium pekinense in fermentation production of lysine

The L-lysine-producing strains, Corynebacterium pekinense CP1008, CP1009 and CP1010 constructed in Example 6 and the original strain AS 1.563 were cultured at the shake flask level and the 3L fermentor level respectively to produce L-lysine as follows.

### (1) Shake flask fermentation:

Corynebacterium pekinense CP1008, CP1009, CP1010 and AS1.563 were inoculated in 500 ml Erlenmeyer flasks containing 50 ml of the seed medium described below, and cultured with shaking at 220 rpm for 8-9h at 30°C. Then, 5 ml of each seed culture solution was inoculated into a 500 ml baffled bottle containing 50 ml of the fermentation medium described below, and cultured with shaking at 220 rpm for 42-46h at 37°C. Concentrated ammonia water was intermittently supplemented to control the pH of the fermentation broth between 7.0 and 7.2. According to the residual sugar, glucose mother liquor with a concentration of 400g/L was added to control the residual sugar of the fermentation broth at 5-10g/L.

### (2) 3L fermentor fermentation:

Corynebacterium pekinense CP1008, CP1009, CP1010 and AS1.563 were inoculated in 1000ml Erlenmeyer flasks containing 100ml of the seed medium described below, and cultured with shaking at 220 rpm for 8-9h at 30°C. Then, each seed culture solution was inoculated into a 3L fermentor containing 900 ml of the fermentation medium described below, and cultured under the pressure of 0.01 MPa for 42-46h at 37°C. The fermentor used is a 3L fermentor: equipped with a built-in constant-speed programmable control pump, which can achieve constant-speed feeding. During the fermentation process, 600g/L glucose was supplemented by a peristaltic pump to control the concentration of glucose in the fermentation system at 5-10g/L, and the fermentation temperature was maintained at 30°C by virtue of a heating jacket and cooling water; the air was supplied to provide dissolved oxygen, and the rotation speed and dissolved oxygen signal were cascaded to control the dissolved oxygen at 30%; concentrated ammonia was supplemented to adjust the pH at about 6.9. The fermentation continued for 52h.

The seed medium and fermentation medium are as follows:

### Seed medium (pH 7.0)

20g of sucrose, lOg of peptone, 5g of yeast extract, 3.5g of urea, 4g of monopotassium phosphate, 10g of dipotassium phosphate, 0.5g of magnesium sulfate heptahydrate, 0.2mg of biotin, 1.5mg of vitamin B1, 2mg of calcium dextrose, and 3 mg of nicotinamide (dissolved in 1L of distilled water).

### Production of medium: pH7.0

40g of glucose, 20g of molasses, 0.4g of phosphoric acid, 15g of ammonium sulfate, 0.87g of magnesium sulfate heptahydrate, 0.88mg of biotin, 6.3mg of vitamin B1, 6.3mg of calcium dextropantothenate, and 42mg of nicotinamide (dissolved in 1L of distilled water).

After the cultivation was completed, HPLC analysis was performed to determine the content of L-lysine produced by the strains. The concentrations of L-lysine in Corynebacterium pekinense CP1008, CP1009, CP1010 and AS1.563 cultures were shown in Table 10.

**Table 10**

| Lysine (g/L) | EPCG1008 | EPCG1009 | EPCG1010 | AS1.563 |
|---|---|---|---|---|
| Shake flask fermentation | 21.23±0.28 | 20.40±0.15 | 9.27±0.37 | 13.98±0.81 |
| 3L Fermentor | 39.65±2.23 | 28.98±1.43 | 14.98±0.56 | 21.26±1.31 |
| fermentation | | | | |

As can be seen from the table above, the transformed strains showed significant differences, both at the shake flask level and at the 3L fermentor level.

The difference trend between shake flask fermentation and fermentor fermentation remains the same. That is, after the expression of asparaginase gene was enhanced, the production of lysine was increased. Correspondingly, after the gene was knocked out, the production of lysine was decreased significantly.

In terms of fermenter acid production data, compared with AS 1.563, CP1008's lysine production was increased by 86.6%; compared with AS1.563, CP1009's lysine production was increased by 36.3%. Compared with AS1.563, CP1010's lysine production was decreased by 29.5%.

It should be noted that the above-described examples are merely illustrative of the invention and are not intended to limit the implementations. Other variations or modifications of the various forms may be made by those skilled in the art in light of the above description. There is no need and no way to exhaust all of the implementations. Obvious changes or variations resulting therefrom are still within the scope of the invention.

## Claims

1. A recombinant bacterium capable of producing L-lysine, wherein the recombinant bacterium has increased expression and/or activity of asparaginase compared to an original bacterium, and the original bacterium refers to a strain capable of accumulating lysine.

2. The recombinant bacterium according to claim 1, wherein the recombinant bacterium has at least two copies of asparaginase encoding gene, and/or the expression of the asparaginase encoding gene of the recombinant bacterium is mediated by a regulatory element with high transcription or high expression activity;
preferably, the regulatory element is a strong promoter;
more preferably, the strong promoter is a P_{tuf} promoter.

3. The recombinant bacterium according to claim 1, wherein the recombinant bacterium has reduced expression and/or activity of homoserine dehydrogenase compared to the original bacterium;
preferably, the reduced expression of homoserine dehydrogenase is achieved in at least one of the following ways: (A) the homoserine dehydrogenase encoding gene of the recombinant bacterium is inactivated, and (B) the expression of the homoserine dehydrogenase encoding gene of the recombinant bacterium is mediated by a regulatory element with low transcription or low expression activity;
the reduced activity of homoserine dehydrogenase is achieved by mutating the 59th valine of homoserine dehydrogenase of the recombinant bacterium to alanine, preferably, the omoserine dehydrogenase encoding gene of the recombinant bacterium is SEQ ID NO.1.

4. The recombinant bacterium according to claim 1, wherein the recombinant bacterium has increased expression and/or activity of pyruvate carboxylase compared to the original bacterium;
preferably, the increased expression of pyruvate carboxylase is achieved by at least one of the following ways: (C) the recombinant bacterium has at least two copies of pyruvate carboxylase encoding gene, and (D) the expression of the pyruvate carboxylase encoding gene of the recombinant bacterium is mediated by a regulatory element with high transcription or high expression activity;
the increased activity of pyruvate carboxylase is achieved by mutating the 458th proline of the pyruvate carboxylase of the recombinant bacterium to serine, preferably, the pyruvate carboxylase encoding gene of the recombinant bacterium is SEQ ID NO.8.

5. The recombinant bacterium according to claim 1, wherein the recombinant bacterium has reduced expression and/or activity of phosphoenolpyruvate carboxykinase compared to the original bacterium; preferably, the phosphoenolpyruvate carboxykinase encoding gene of the recombinant bacteria is inactivated, and/or the expression of the phosphoenolpyruvate carboxykinase encoding gene is mediated by a regulatory element with low transcription or low expression activity;
more preferably, the inactivated is knocking out the phosphoenolpyruvate carboxykinase encoding gene of the recombinant bacterium.

6. The recombinant bacterium according to claim 1, wherein the recombinant bacterium has increased expression and/or activity of dihydropyridine dicarboxylate reductase (*dapB*) compared to the original bacterium;
preferably, the recombinant bacterium has at least two copies of dihydropyridine dicarboxylate reductase encoding gene, and/or the expression of the dihydropyridine dicarboxylate reductase encoding gene is mediated by a regulatory element with high transcription or high expression activity;
more preferably, the regulatory element is a strong promoter;
most preferably, the strong promoter is a P_{tuf} promoter of the original bacterium.

7. The recombinant bacterium according to claim 1, wherein the recombinant bacterium has increased expression and/or activity of aspartate kinase, diaminopimelate dehydrogenase and/or diaminopimelate decarboxylase compared to the original bacterium;
preferably, the recombinant bacterium has at least two copies of aspartate kinase encoding gene, diaminopimelate dehydrogenase encoding gene and/or diaminopimelate decarboxylase encoding gene, and/or the expression of the aspartate kinase encoding gene, the diaminopimelate dehydrogenase encoding gene and/or the diaminopimelate decarboxylase encoding gene is mediated by a regulatory element with high transcription or high expression activity;
more preferably, the regulatory element is a strong promoter;
most preferably, the strong promoter is a P_{tuf} promoter of the original bacterium.

8. The recombinant bacterium according to any of claims 1-7, wherein the original bacterium is a bacterium selected from Corynebacterium, Brevibacterium, Bacillus, Bifidobacterium, and Lactobacillus or a fungus selected from yeast;
preferably, the bacterium of Corynebacterium is selected from Corynebacterium glutamicum, Corynebacterium pekinense, Corynebacterium efficiens, Corynebacterium crenatum, Corynebacterium thermoaminogenes, Corynebacterium aminogenes, Corynebacterium lilium, Corynebacterium callunae, and Corynebacterium herculis;
the bacterium of Brevibacterium is selected from Brevibacteriaceae flvum, Brevibacteriaceae lactofermentum and Brevibacteriaceae ammoniagenes;
the bacterium of Bacillus is selected from Bacillus licheniformis, Bacillus subtilis and Bacillus pumilus;
the bacterium of Bifidobacterium is selected from Bifidobacterium bifidum, Bifidobacterium longum, Bifidobacterium breve, and Bifidobacterium adolescentis;
the bacterium of Lactobacillus is one of Lactobacillus acidophilus, Lactobacillus casei, Lactobacillus delbrueckii subsp and Lactobacillus fermentum;
the fungus of yeast is selected from Candida utilis, Saccharomyces cerevisiae, Pichia pastoris and Hansenula polymorpha.

9. A construction method of the recombinant bacterium according to any of claims 1-8, comprising the following step:
increasing the expression and/or activity of asparaginase in an original bacterium,
wherein preferably, the increasing the expression and/or activity of the asparaginase in the original bacterium is achieved by at least one of: (E) increasing the copy number of asparaginase encoding gene in the original bacterium, and (F) replacing a regulatory element for the asparaginase encoding gene in the original bacterium with a regulatory element with high transcription or high expression activity.

10. A production method of L-lysine, comprising the following step:
fermenting and culturing the recombinant bacterium according to any of claims 1-8.
